# EUROPEAN PATENT SPECIFICATION

(11) **EP 2 255 679 B1**
(45) Date of publication and mention of the grant of the patent: **19.10.2016**
(21) Application number: 08784142.5
(22) Date of filing: 26.08.2008
(51) Int. Cl.: A23L 33/145, A23L 31/15, A23L 27/23, C12P 21/06

(54) **YEAST EXTRACT INCLUDING DISODIUM INOSINATE SALT AND DISODIUM GUANYLATE SALT AND PRODUCING METHOD THEREOF**
HEFEEXTRAKT, DER DINATRIUMINOSINAT-SALZ UND DINATRIUMGUANYLAT-SALZ ENTHÄLT, SOWIE VERFAHREN ZU SEINER HERSTELLUNG
EXTRAIT DE LEVURE COMPORTANT DE L'INOSINATE DISODIQUE ET DU GUANYLATE DISODIQUE ET SON PROCÉDÉ DE PRODUCTION

(30) Priority: 19.02.2008 CN 200810007940
(43) Date of publication of application: 01.12.2010
(73) Proprietor: Angel Yeast Co., Ltd, Hubei 443003 (CN)
(72) Inventor: YU, Xuefeng, Yichang Hubei 443003 (CN); LI, Zhihong, Yichang Hubei 443003 (CN); YU, Minghua, Yichang Hubei 443003 (CN); YAO, Juan, Yichang Hubei 443003 (CN); LI, Pei, Yichang Hubei 443003 (CN); LI, Ku, Yichang Hubei 443003 (CN); TANG, Guanqun, Yichang Hubei 443003 (CN)
(74) Representative: Gerbino, Angelo
(86) International application number: PCT/CN2008/072153
(87) International publication number: WO 2009/103205

(56) References cited:
- WO-A2-2005/067734
- CN-A- 1 481 721
- CN-A- 1 806 653
- CN-A- 1 961 743
- US-A- 5 288 509
- CONWAY J ET AL: "THE EFFECT OF THE ADDITION OF PROTEASES AND GLUCANASES DURING YEAST AUTOLYSIS ON THE PRODUCTION AND PROPERTIES OF YEAST EXTRACTS", CANADIAN JOURNAL OF MICROBIOLOGY, NRC RESEARCH PRESS, CA, vol. 47, no. 1, 1 January 2001 (2001-01-01), pages 18-24, XP009044657, ISSN: 0008-4166, DOI: DOI:10.1139/CJM-47-1-18
- YAN,ZHIYUN ET AL.: 'Natural Flavouring Agents-Yeast Extract.' FOOD AND FERMENTATION INDURSTRIES. vol. 24, no. 6, June 1998, pages 42 - 48, XP008140898

## Description

### Technical field

The present invention relates to preparative method of a yeast extract containing 4-30% of disodium inosinate salt and disodium guanylate salt (I+G, wherein I stands for disodium inosinate salt and G for disodium guanylate salt), and the yeast extract product containing disodium inosinate salt and disodium guanylate salt prepared thereby.

### Background

Yeast extract is a condensate of soluble nutrients and flavor substances that can be absorbed directly by human bodies, and is prepared by degradating proteins in yeast cells into amino acids and polypeptides, nucleic acids into nucleotides, and extracting them from yeast cells together with other effective components, such as vitamin B, glutathione and microelements.

In recent years, as the improvement of people's living standard, new requirements for the content of diet have been raised. Various foods are developed to becoming more and more natural, convenient, delicious and multi-functional. And food condiments have been changed from the previously used brewing or chemical ones into natural seasoning complexes with advanced taste. Meanwhile, condiments in the market are being developed into advanced seasoning complexes that are delicious, convenient, natural and nutrient. Yeast extract contains amino acids and polypeptides as well as other effective components, like nucleotides, vitamins, organic acids and minerals. With strong and delicious flavor and taste of meat, the yeast extract is an advanced food seasoning that has multi-function of nutrient, seasoning and health-care, thus becoming more and more popular among customers.

The global demand in yeast extract market is estimated as about 1.5 billion dollars. Some food additive enterprises in Europe, such as DSM, are ahead of this field and provide 2/3 of products on the market. Growth on the market is still strong, reflecting demands for natural and efficient flavor potentiators.

Yeast extract is widely used in catering industry for the adjustment as required of flavor, acidity and feeling of chewing, etc. In cases of applications, it also affects taste of cayenne and meat, thus providing possibilities of reducing ingredient cost or enhancing flavor.

Recently, the use of yeast extract is significantly expanded with the development of biotechnology. The newest generation of yeast extract contains abundant glutamic acid and nucleotide, further improving its capacity as flavor potentiator. Together with amino acids and polypeptides that existed before, it can significantly reduce the content of sodium salts while not losing the original flavor. More importantly, it allows the processing trademark to be marked with natural product, thus preventing anxieties of artificial synthesis or chemical additives from customers.

Since yeast extract has special seasoning and nutrient effect in food processing, it is widely used in developed countries such as Europe, America and Japan, and its ratio in flavor seasoning market demand is up to 35%. Taking Japan for an example, besides the part domestically produced, the yeast extract is imported in large amounts from abroad, and the consumption increases year by year. As a food seasoning, yeast extract has been widely used in instant noodles, meat products and condiments.

Disodium 5'-ribonucleotide is a nucleotide-based food fresh enhancer, which is a mixture of 5'-disodium inosinate (IMP) and 5'-disodium guanylate (GMP) in the ratio of 1:1 and can be added directly into foods to improve their fresh flavor. With comparatively low cost and best effect, it is one of the main flavor development ingredients of flavoring bag of instant noodles as well as condiments such as chicken essence, chicken powder and fresh-enhanced soy sauce. Being honored as "powerful gourmet powder", it is used with sodium glutamate (MSG) mixed together, with an amount of about 2%-5% by weight of MSG.

A process for preparing yeast extract with ultra-low salt content, light color and meaty smell was disclosed (ZHONG Rui-min; HUANG Guo-qing; LIU Jian-nan, Dept. of Food Technology; Yingdong School of Biotechnology; Shaoguan University; China Condiment, issue No. 2, 2004), wherein fresh beer yeast cell wall was able to be broken to a certain extent after cells were washed with cold water to remove bitterness before being slowly frozen and thawed, and 46.3% by weight of gross nitric substances in the cell was extravasated by ultrasonic wall broken treatment with the addition of 5% of alcohol. The brown color of hydrolyzed solution was enhanced greatly with its high content of glucose by weight being 1.49%, (fresh yeast basis) before autolyzing process. The slight yellow autolyzed solutions were obtained by pre-treatment of glucose-eliminating processes of dry wine yeast fermentation and glucose oxidase which both contributed to nice color-keeping effect on solutions. The fermentation pretreatment also changed the flavor of yeast extract markedly, with the extract possessing chicken smell and high nitrogen content in the form of amino acids (5.12%, dry yeast basis). With above procedures, a kind of yeast extract with properties of ultra-low salt content, slight color and meaty smell can be obtained.

Another process for preparing disodium 5'-ribonucleotide I+G through double enzyme degradation of nucleic acid (Sugarcane and Canesugar, issue No. 3, 2000) was also disclosed. With yeast nucleic acid (RNA) as raw material, enzymes were prepared with submerged fermentation of industrialized production with modern bioengineering technology, and two steps of enzymatic degradation was conducted. The resulting substance was separated, purified, dried and grinded, and a compound food freshener comprising 5'-sodium inosinate (5'-IMP.Na₂.7H₂O, called "I") and 5'-sodium guanylate (5'-GMP.Na₂.7H₂O, called "G"), each for about 50%, was obtained.

Another yeast extract with high 5'-ribonucleotide content and preparation thereof (Chinese Application No. 200510124942.1) was disclosed. The application provided a yeast extract with higher 5'-ribonucleotide and amino acid content, and the preparative process thereof. Food yeast was treated with acidic aqueous solution and separated by centrifugation. After being washed with water, the resulting precipitate was contacted with enzymes produced by *Actinomyces*, and yeast extract with high 5'-ribonucleotide content was obtained with 5'-inosinate and 5'-guanylate of at least 24wt% in total, peptides of at least 20wt%, and peptides and free amino acids of at least 28wt% in total.

Ordinary yeast extract contains plenty of proteins and amino acids with mellow taste, but with less disodium 5'-ribonucleotide content, it possesses insufficient fresh flavor. And, pure disodium 5'-ribonucleotide costs higher and while being used alone, turns to show less mellow taste and insufficient fresh flavor, thus being obligated to be mixed together with amino acids to generate fresh flavor. However, in the mixed manner wherein the disodium ribonucleotide was added as food additives, the product must be labeled as "additives contained", thus being deprived of its natural and non-additive characteristics.

US-5 288 509 discloses a method for preparing a yeast extract by enzymatic treatment, and the yeast extract obtainable by this method. 300 g of a yeast suspension, obtained by heating 220g of baker's yeast in 80g of water at about 100°C for 30 min at pH 6 and cooling at 65°C, was mixed with 30 g of a malt rootlets suspension, obtained by heating 2.7 g (milled) malt rootlets in 27g of water containing 24 mg of zinc acetate, and 109 mg papain. The mixture was saturated with oxygen. Subsequently, the mixture was heated for 18 hours at 65°C at pH 5.6. The mixture was filtered. The product was obtained by evaporation of the filtrated containing 3.90% by weight 5'-GMP, yeast extract yield : 46% by weight.

WO-2005/067 734 discloses a process to produce a composition containing 5'-ribonucleotides, and the compositions containing 5'-ribonucleotides. 2 1 of cream yeast from Saccharomyces cerevisiae was warmed up to 60 °C. Subsequently 0.4 ml Pescalase was added and the mixture was incubated for 4 hours at pH 6.0 and 60 °C. The conditions were adjusted to pH 5.1 and 51.5°C and an additional 2ml of Pescalase was added to the reaction mixture. The mixture was incubated for 20 hours at pH 5.1, 51.5°C. Next, the mixture or autolysate was heated for 1 hour at 65°C to inactivate all enzyme activity. The extract was separated from the insoluble cell walls by means of centrifugation. The resulting cell wall fraction was treated with 5'-phosphodiesterase to hydrolyze the RNA into 5'-ribonucleotides at a temperature of 65°C and a pH of 5.3. Next the 5'-AMP was converted by the enzyme deaminase into 5'-IMP at a temperature of 55°C and at pH5.1. Finally, the 5'-ribonucleotides were separated from the insoluble cell wall fraction by means of centrifugation.

Therefore, there is need to provide a yeast extract with high disodium 5'-ribonucleotide content, while being naturally generated and containing no additive.

### Disclosure of the present invention

Therefore, the aim of the present invention is to provide a product that is rich in disodium ribonucleotide and possesses the combined advantages of yeast extract and disodium 5'-ribonucleotide. More importantly, being natural and non-additive, the product can allow trademarks to be labeled as "inartificial", thus preventing anxieties for artificial synthesis or chemical additives from customers.

In one aspect, the present invention provides a method of producing a yeast extract containing 4-30% I+G (disodium inosinate salt and disodium guanylate salt), comprising:
a) Providing at least a kind of yeast with ribonucleic acid content of 8wt% or more which is selected from group consisting of baker's yeast, fermented *Saccharomyces cerevisiae* and *Torula,* to prepare yeast cream;
b) Sterilizing the yeasts in said yeast cream under the temperature of 40-95°C;
c) Centrifuging the yeast cream with yeasts sterilized to obtain supernatant;
d) Adding protease, nuclease and transaminase into the supernatant and carring out enzymolysis under conditions of pH of 4.0-6.8, temperature of 35-80°C for 12-25 hours;
e) After said enzymolysis, deactivating the enzymes in the supernatant with heat treatment at 75-90°C for 50-70 minutes;
f) Concentrating said supernatant with deactivated enzymes into 35-40wt%, and spray-dring the resulting dry substance to obtain yeast extract with higher disodium inosinate salt and disodium guanylate salt content; and with the final content of disodium inosinate salt and disodium guanylate salt in yeast being 4-30% in total.

According to the method of the present invention, enzymes are added with a ratio of 1:1:1 in step (d).

According to the method of the present invention, enzymes are added with an amount of 0.1-0.3% by weight in dry substance of supernatant in step (d).

According to the method of the present invention, added enzymes functions preferably in temperature of 45-68°C.

According to the method of the present invention, pH is controlled in the scope of 4.5-6.8 in step (e).

According to the method of the present invention, the reaction time is controlled between 16-20 hours in step (e).

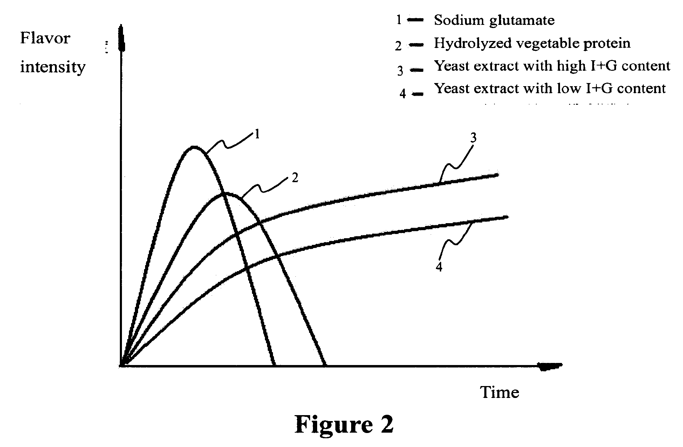

The yeast extract prepared according to methods of the present invention possesses more delicious mouthfeel, enhancing its capacity to improving food taste. Mouthfeel, being a subjective assessment according to different human beings, has no unified international standard to evaluate by far. It could be seen from figure 2, that the flavor intensity of yeast extracts with higher content turns to last longer. Although adding nucleotide directly into ordinary yeast extract can also enhance the fresh flavor, but it arouse non-natural problem.

### Instruction of figures

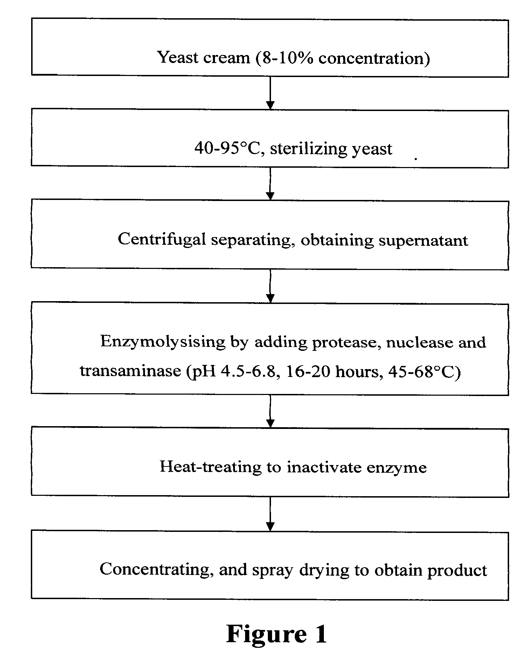

Figure 1 is a process flow chart of one embodiment according to the present invention.
Figure 2 is a curve diagram of mouthfeel intensity versus time of different substances.

### Embodiments

Detailed description yeast extract production process according to the present invention will be made as following in accordance with figures. Those skilled in the art should understand that the detailed description as following aims at facilitating the understanding of the present invention, instead of limiting the protection scopes of the present invention.
a) According to processes of the present invention, baker's yeast with ribonucleic acid (RNA) content of 8wt% or more which were cultured specially with molasses as culture medium can be used. Residuary yeast after beer fermentation, or *Torula* also can be used, wherein ribonucleic acid content in these yeast should achieve 8wt% or more, too.
b) The yeasts in yeast cream are sterilized under the temperature of 40-95°C;
c) The yeast cream with yeasts sterilized is centrifuged to obtain supernatant;
d) Protease, nuclease and transaminase of 0.1-0.3% by weight are added into the supernatant with the ratio of 1:1:1 and enzymolysis is carried out under conditions of pH of 4.0-6.8, temperature of 35-80°C for 12-25 hours;
e) The enzymes in the supernatant are deactivated after enzymolysis with heat treatment at 75-90°C, and the supernatant is centrifuged;
f) The supernatant with inactivated enzymes is concentrated and the resulting dry substance is spray-dried to obtain paste yeast extract with higher disodium inosinate salt and disodium guanylate salt content; and the total content of disodium inosinate salt and disodium guanylate salt in yeast is 4-30%.

According to the methods of the present invention, the enzymes which were added in step (d) functions in temperature of 45-68°C.

### Spray drying procedure:

The supernatant is spray-dried after being concentrated to about 30% of dry substance content, then a yeast extract with high I+G content (4-30%) is obtained.

The present invention is further illustrated with examples as follows.

### Example

### Example 1

### Producing yeast extract

First, yeast cream was prepared by providing baker's yeast with 8 wt% of ribonucleic acid content, then yeasts in said yeast cream were sterilized under the temperature of 50°C and said yeast cream was centrifuged to obtain supernatant. Next, protease, nuclease and transaminase in the total amount of 0.2% by weight were added into the supernatant with the ratio of 1:1:1 and enzymolysis was carried out under conditions of pH of 5.2, temperature of 45°C for 16 hours. Enzymes in the supernatant were deactivated after said enzymolysis with heat treatment at 85°C for 30 minutes. At last, said supernatant with deactivated enzymes was concentrated and spray-dried in a spray-drying tower to obtain yeast extract with combined content of disodium inosinate salt and disodium guanylate salt of 18%, wherein disodium inosinate salt accounted for 10% and disodium guanylate salt accounted for 8%. Said content was determined by HPLC respectively. Since the present application aimed at preparing products of combined I and G, it was unnecessary to make separations in the final product.

### Yeast extract

The yeast extract with high I+G content of the present invention possessed more delicious mouthfeel, enhancing its capacity to improving food taste.

The inventor operated similarly as above mentioned and altered the parameters of operation conditions and the amount of each material. Conditions listed as following were found to be essentially suitable for the practice of the present invention. And yeast extract prepared in preferred conditions had essentially the same effect with product of example 1.

Examples 2-10 were carried out with the same steps as above mentioned, using technological conditions listed in table 1 and 2.

**Table 1**

| | | Example 2 | Example 3 | Example 4 | Example 5 | Example 6 | Example 7 |
|---|---|---|---|---|---|---|---|
| Ribonucleic acid content in yeast cream (%) | | 8 | 8 | 9 | 9 | 10 | 10 |
| Yeast-sterilizing temperature (°C) | | 40 | 45 | 50 | 55 | 60 | 65 |
| PH of yeast cream | | 4.5 | 4.9 | 5.4 | 5.9 | 6.4 | 6.8 |
| Enzymolysis temperature (°C) | | 35 | 45 | 55 | 65 | 75 | 80 |
| Heat treat ment | Temperature (°C) | 85 | 85 | 85 | 85 | 85 | 85 |
| | Time (min) | 30 | 30 | 30 | 30 | 30 | 30 |
| | PH | 6.5 | 6.5 | 6.5 | 6.5 | 6.5 | 6.5 |
| I+G content (%) | | 7.4 | 10 | 12 | 14 | 17 | 20 |

**Table 2**

| | | Example 8 | Example 9 | Example 10 |
|---|---|---|---|---|
| Ribonucleic acid content in yeast cream (%) | | 10 | 11 | 12 |
| Yeast- sterilizing temperature (°C) | | 70 | 75 | 80 |
| PH of yeast cream | | 4.5 | 4.9 | 5.4 |
| Enzymolysis temperature (°C) | | 35 | 45 | 55 |
| Heat treat ment | Temperature (°C) | 85 | 85 | 85 |
| | Time (min) | 30 | 30 | 30 |
| | PH | 6.5 | 6.5 | 6.5 |
| I+G content (%) | | 23 | 26 | 29 |

The results showed that, according to preparative methods of the present invention, a yeast extract with total content of 4-30% of disodium inosinate and disodium guanylate could be obtained.

### Control example:

Chinese Application No. 200510124942 disclosed a yeast extract with high 5'-ribonucleotide content and preparation method thereof.

In said method, food yeast was treated with acidic aqueous solution and separated by centrifugation. After being washed with water, the resulting precipitate was contacted with enzymes produced by *Actinomyces*, and yeast extract with high 5'-ribonucleotide content was prepared, which contained 24wt% or more 5'-inosinate and 5'-guanylate in total, 20wt% or more peptides and 28wt% or more peptides and free amino acids in total.

The content of I+G in said comparative example was relatively high, while the content of peptides and free amino acids was relatively low.

The content of peptides and free amino acids of examples in the present invention accounted for more than 35%. In addition, there could be higher content of I+G and better mouthfeel. Table 3 shows the results of the examples of the present invention together with comparative example of the prior art.

**Table 3**

| | Example 8 | Example 9 | Comparative example |
|---|---|---|---|
| Content of peptides and free amino acids | 35% | 38% | 28% |

Wherein, ratios of the content of peptides that was determined by high performance liquid chromatography, and content of free amino acids that was determined by amino-acid analyzer, were from 2:3 to 1:1.

## Claims

1. A method for producing a yeast extract containing disodium inosinate salt and disodium guanylate salt, comprising:
a) providing at least a kind of yeast with ribonucleic acid content of 8wt% or more which is selected from groups consisting of baker's yeast, fermented *Saccharomyces cerevisiae*, and *Torula,* to prepare yeast cream;
b) sterilizing the yeast in said yeast cream under temperature of 40-95°C;
c) centrifuging said yeast cream to obtain supernatant;
d) adding protease, nuclease and transaminase into said supernatant simultaneously, and carrying out enzymolysis under conditions of pH of 4.0-6.8, temperature of 35-80°C for 12-25 hours;
e) after said enzymolysis, deactivating the enzymes in the supernatant with heat treatment at 75-90°C for 50-70 minutes;
f) concentrating said supernatant with deactivated enzymes into 35-40wt%, and spray-drying the resulting dry substance to obtain yeast extract with 4-30% content of disodium inosinate salt and disodium guanylate salt.

2. The method according to claim 1, **characterized in that** said enzymes which are added in said step (d) functions at the temperature of 45-68°C.

3. The method according to claim 1 or 2, **characterized in that** said enzymes are added with a ratio of 1:1:1 each other in said step (d).

4. The method according to claim 1 or 2, **characterized in that** said enzymes in dry substance of supernatant are added with an amount of 0.1-0.3% by weight in said step (d).

5. The method according to claim 1, **characterized in that** pH is controlled in the scope of 4.5-6.8 in step (d).

6. The method according to claim 1, **characterized in that** the reaction time is controlled between 16-20 hours in step (d).

## Patentansprüche

1. Ein Verfahren zum Herstellen eines Hefeextrakts, der Dinatriuminosinatsalz und Dinatriumguanylatsalz enthält, das folgende Schritte aufweist:
a) Bereitstellen zumindest einer Hefeart mit einem Ribonucleinsäuregehalt von 8 Gew.-% oder mehr, die aus Gruppen ausgewählt ist, die Bäckerhefe, fermentierte *Saccharomyces cerevisiae* und *Torula* umfassen, um Hefecreme vorzubereiten;
b) Sterilisieren der Hefe in der Hefecreme bei einer Temperatur von 40-95°C;
c) Zentrifugieren der Hefecreme, um einen Überstand zu erhalten;
d) Gleichzeitiges Hinzufügen von Protease, Nuklease und Transaminase zu dem Überstand und Ausführen einer Enzymolyse unter Bedingungen eines pH-Werts von 4,0-6,8, einer Temperatur von 35-80°C für 12-25 Stunden;
e) Deaktivieren der Enzyme in dem Überstand nach der Enzymolyse mit einer Wärmebehandlung bei 75-90°C für 50-70 Minuten;
f) Konzentrieren des Überstands mit deaktivierten Enzymen auf 35-40 Gew.-% und Trocknen der resultierenden Trockensubstanz im Sprühverfahren, um einen Hefeextrakt mit einem Dinatriuminosinatsalz- und Dinatriumguanylatsalzgehalt von 4-30 % zu erhalten.

2. Das Verfahren gemäß Anspruch 1, **dadurch gekennzeichnet, dass** die Enzyme, die bei Schritt (d) hinzugefügt werden, bei der Temperatur von 45-68°C wirken.

3. Das Verfahren gemäß Anspruch 1 oder 2, **dadurch gekennzeichnet, dass** bei Schritt (d) die Enzyme mit einem Verhältnis zueinander von 1:1:1 hinzugefügt werden.

4. Das Verfahren gemäß Anspruch 1 oder 2, **dadurch gekennzeichnet, dass** bei Schritt (d) die Enzyme in Trockensubstanz des Überstands in einer Menge von von 0,1-0,3 Gewichtsprozent hinzugefügt werden.

5. Das Verfahren gemäß Anspruch 1, **dadurch gekennzeichnet, dass** bei Schritt (d) der pH-Wert in den Bereich von 4,5-6,8 geregelt wird.

6. Das Verfahren gemäß Anspruch 1, **dadurch gekennzeichnet, dass** bei Schritt (d) die Reaktionszeit auf zwischen 16 und 20 Stunden geregelt wird.

## Revendications

1. Procédé pour la production d'un extrait de levure contenant du sel inosinate disodique et du sel guanylate disodique, comprenant :
a) la fourniture d'au moins une sorte de levure avec une teneur en acide ribonucléique de 8 % en poids ou supérieure qui est choisie dans des groupes constitués de la levure de boulanger, *Saccharomyces cerevisiae* fermentée, et *Torula,* pour préparer une crème de levure ;
b) la stérilisation de la levure dans ladite crème de levure sous une température de 40 à 95°C ;
c) la centrifugation de ladite crème de levure pour obtenir un surnageant ;
d) l'ajout d'une protéase, d'une nucléase et d'une transaminase dans ledit surnageant simultanément, et la réalisation d'une enzymolyse dans des conditions de pH de 4,0 à 6,8, à une température de 35 à 80°C pendant 12 à 25 heures ;
e) après ladite enzymolyse, la désactivation des enzymes dans le surnageant avec un traitement à la chaleur à une température de 75 à 90°C pendant 50 à 70 minutes ;
f) la concentration dudit surnageant avec les enzymes désactivées à 35 à 40 % en poids, et le séchage par pulvérisation de la substance sèche résultante pour obtenir l'extrait de levure avec une teneur de 4 à 30 % de sel inosinate disodique et de sel guanylate disodique.

2. Procédé selon la revendication 1, **caractérisé en ce que** lesdites enzymes qui sont ajoutées dans ladite étape (d) fonctionnent à la température de 45 à 68°C.

3. Procédé selon la revendication 1 ou 2, **caractérisé en ce que** lesdites enzymes sont ajoutées dans un rapport de 1:1:1 les unes par rapport aux autres dans ladite étape (d).

4. Procédé selon la revendication 1 ou 2, **caractérisé en ce que** lesdites enzymes dans la substance sèche du surnageant sont ajoutées avec une quantité de 0,1 à 0,3 % en poids dans ladite étape (d).

5. Procédé selon la revendication 1, **caractérisé en ce que** le pH est contrôlé dans la plage de 4,5 à 6,8 dans l'étape (d).

6. Procédé selon la revendication 1, **caractérisé en ce que** le temps de réaction est contrôlé entre 16 et 20 heures dans l'étape (d).
